(19) Europäisches Patentamt / European Patent Office / Office européen des brevets

(11) **EP 4 722 765 A1**

(12) **DEMANDE DE BREVET EUROPEEN**

(43) Date de publication:
08.04.2026 Bulletin 2026/15

(21) Numéro de dépôt: 25203787.4

(22) Date de dépôt: 22.09.2025

(51) Classification Internationale des Brevets (IPC):
*G01V 3/12* (2006.01)     *G01S 13/88* (2006.01)
*G01V 3/15* (2006.01)     *G01V 3/38* (2006.01)

(52) Classification Coopérative des Brevets (CPC):
G01V 3/12; G01S 13/885; G01V 3/15; G01V 3/38

(84) Etats contractants désignés:
AL AT BE BG CH CY CZ DE DK EE ES FI FR GB GR HR HU IE IS IT LI LT LU LV MC ME MK MT NL NO PL PT RO RS SE SI SK SM TR
Etats d'extension désignés:
BA
Etats de validation désignés:
GE KH LA MA MD TN

(30) Priorité: 02.10.2024 FR 2410590

(71) Demandeur: Commissariat à l'Energie Atomique et aux Energies Alternatives
75015 Paris (FR)

(72) Inventeurs:
• PAGANI, Pascale
38054 GRENOBLE (FR)
• DORE, Jean-Baptiste
38054 GRENOBLE (FR)
• D'ERRICO, Raffaele
38054 GRENOBLE (FR)

(74) Mandataire: Atout PI Laplace
Immeuble Up On
25 Boulevard Romain Rolland
CS 40072
75685 Paris Cedex 14 (FR)

(54) **METHODE DE DETERMINATION DE PROPRIETES RADIOELECTRIQUES D'UN MILIEU NON-HOMOGENE AU MOYEN D'UN SYSTEME DE DETECTION RADIOFREQUENCE**

(57) Méthode de détermination de propriétés radioélectriques d'un milieu comprenant les étapes de :
Déterminer (101), une mesure d'une fonction de transfert fréquentielle d'un canal de transmission caractérisant ledit milieu,
Déterminer (102) un modèle de ladite fonction de transfert fréquentielle
Déterminer (103,104) une fonction d'estimation d'un coefficient de corrélation entre l'au moins une mesure d'une fonction de transfert fréquentielle et le modèle de ladite fonction,
Fixer (400) une valeur initiale de l'au moins une propriété radioélectrique,
Rechercher (402) un ensemble de premiers maxima locaux de la fonction d'estimation sur le domaine défini par l'au moins une variable de position pour la valeur initiale de l'au moins une propriété radioélectrique,
Pour chaque premier maximum local, rechercher (403) un second maximum local de la fonction d'estimation sur le domaine défini par l'au moins une variable caractérisant l'au moins une propriété radioélectrique du milieu et sur un domaine défini par l'au moins une variable de position contraint par le premier maximum local.

[Fig. 4]

FIG.4

EP 4 722 765 A1

## Description

L'invention concerne le domaine des dispositifs et méthodes non destructives permettant de caractériser la composition d'un matériau ou d'un milieu non homogène au moyen d'un système radiofréquence. Par exemple, l'invention concerne notamment les radars à pénétration de sol qui permettent de détecter, localiser ou identifier des cibles souterraines. L'invention peut concerner également le domaine de la santé, par exemple la caractérisation de tissus biologiques.

L'invention porte plus précisément sur une méthode de détermination de propriétés radioélectriques d'un milieu non homogène au moyen d'un système radiofréquence. La méthode proposée permet une résolution avec une complexité diminuée compatible d'une implémentation embarquée sur un dispositif à ressources limitées.

Pour toutes les applications qui visent à détecter des éléments dans un milieu ou un matériau à partir d'un système de détection radiofréquence, la connaissance des propriétés radioélectriques du milieu de détection est importante.

En effet, la localisation spatiale des cibles recherchées est généralement réalisée par l'analyse du temps de propagation des signaux radioélectriques émis et reçus par le système radiofréquence, qui sont ensuite convertis en distances à l'aide d'une estimation de la vitesse de propagation des ondes radioélectriques dans le milieu. Or, la vitesse de propagation d'une onde électromagnétique dépend des propriétés radioélectriques du milieu, et en particulier (bien que pas exclusivement) de sa permittivité diélectrique. Les propriétés radioélectriques du milieu permettent de décrire la réponse de ce milieu à un champ électrique appliqué.

Par exemple, dans le cas de radars à pénétration de sol, le milieu traversé par les ondes électromagnétiques est en général le sol. Pour le cas d'applications dans le domaine de la santé, le milieu traversé concerne un ensemble de tissus biologiques.

La plupart du temps, des hypothèses a priori sont faites sur les valeurs effectives des caractéristiques radioélectriques du milieu telles que la permittivité diélectrique. Or la précision de ces valeurs peut être importante pour obtenir une précision suffisante pour la détection et la caractérisation de cibles.

L'invention répond donc à un problème général de caractérisation précise des propriétés radioélectriques d'un milieu dans lequel une onde électromagnétique se propage, notamment la permittivité diélectrique ou la perméabilité.

Par ailleurs, un autre problème général dans ce contexte consiste à réaliser cette caractérisation avec une complexité de traitement limitée permettant l'intégration de la méthode dans un dispositif embarqué.

Dans le domaine des radars à pénétration de sol, il existe plusieurs méthodes de détection de la position d'une cible.

Une méthode de l'état de l'art connue est l'ajustement de courbe hyperbole décrit par exemple dans la référence [1]. Lorsqu'une antenne du radar à pénétration de sol est déplacée au-dessus de la surface du sol et que l'onde pénétrante rencontre des cibles enterrées ou des interfaces entre différentes couches du sol, le signal reçu observé dans le domaine temporel présente une forme d'hyperbole, en raison des temps de parcours des ondes réfléchies, qui diffèrent suivant la position relative des antennes du radar et de la cible. Plusieurs configurations de positions d'antennes peuvent être utilisées afin d'observer ce phénomène. Par exemple, les antennes d'émission (Tx) et de réception (Rx) peuvent se déplacer d'un mouvement identique, l'une restant ainsi en position fixe par rapport à l'autre. Alternativement, l'antenne Tx peut réaliser un déplacement symétrique à celui de l'antenne Rx par rapport à un point central fixe, cette configuration étant alors nommée « Common Mid Point ». Enfin, une antenne peut se placer à différentes positions par rapport à la seconde antenne fixe, dans une configuration appelée « Wide Angle Reflection and Refraction ».

Toutes ces techniques d'observation permettent d'obtenir un diagramme en forme d'hyperbole lorsqu'on observe le signal reçu dans le domaine temporel en fonction d'une métrique de position des antennes. Le sommet de l'hyperbole indique alors la position effective de la cible (ou de l'interface) et la forme de l'hyperbole dépend du pas horizontal et de la vitesse du signal dans le matériau : plus la vitesse est élevée, plus l'hyperbole est large et inversement. En analysant la forme de l'hyperbole, on peut remonter à la vitesse de propagation de l'onde dans le milieu et ainsi remonter à la permittivité électrique du milieu. L'ajustement du signal observé sur une hyperbole théorique est réalisé par une analyse de semblance, telle que décrite par exemple dans les références [2, 3].

Cette méthode est très courante pour l'étalonnage de données GPR, mais présente plusieurs inconvénients majeurs. La précision de la méthode reste faible en raison de la difficulté d'ajuster une courbe théorique sur une observation expérimentale entachée d'incertitude. Augmenter la précision nécessite d'acquérir un plus grand nombre d'observations, ce qui implique un déplacement des antennes à chaque observation. Le déplacement des antennes peut être évité si on dispose de plusieurs antennes Tx et Rx réparties spatialement, mais dans ce cas la précision de la technique d'ajustement est faible. La méthode est également peu adaptée à la détection de couches stratifiées pour un milieu non homogène, car les différentes réflexions produisent des hyperboles qui se superposent, rendant l'ajustement plus difficile. Cette situation nécessite de mettre en œuvre des algorithmes complexes, comme l'illustre la référence [4].

Un autre type de méthode connue concerne les méthodes de migration, telles que présentées par exemple dans la référence [5]. Ces méthodes cherchent à réduire l'imprécision de la méthode d'ajustement d'hyperboles due aux incertitudes d'observation du signal reçu. Il s'agit d'une forme de traitement mathématique dont le but principal est d'augmenter la précision des traces hyperboliques des cibles et interfaces. Théoriquement, ce processus permet de réduire les hyperboles acquises par le radar en un point unique de localisation. Cependant, la mise en œuvre de cette technique s'avère délicate lorsqu'on l'applique à des données expérimentales potentiellement bruitées. Il existe une grande variété de méthodes de migration différentes parmi lesquelles la sommation hyperbolique, la migration de Kirchhoff, la focalisation en projection inverse, la migration en décalage de phase, et la migration $\omega$-$k$ [5].

Le principal paramètre requis pour réduire l'hyperbole à un point correspond aux propriétés diélectriques du sol tel qu'enseigné dans la référence [6]. Cela fait du processus de migration un moyen d'augmenter la précision de l'ajustement de la courbe puisque le processus ne fonctionne correctement que si la vitesse de transmission au sol appliquée est précise. Ainsi les méthodes de migration utilisent une estimation moyenne de la permittivité relative d'un matériau pour une profondeur donnée. Un inconvénient de ces méthodes est leur relative imprécision lorsque le matériau est composé de plusieurs couches de permittivités différentes.

La référence [6] propose une méthode appliquée à la détection de cibles enterrées. Elle propose de tester différentes valeurs de permittivités moyennes pour une acquisition donnée. La permittivité sélectionnée est celle qui minimise la surface de la cible détectée. De ce fait, cette méthode n'est pas générique et n'est pas applicable en l'absence de cible, par exemple dans le cas d'un matériau inhomogène stratifié. D'autre part, la métrique de la surface apparente de la cible est peu précise et n'est adaptée qu'aux cibles de forme simple, dont la surface apparente varie de manière monotone avec l'erreur absolue dans la permittivité supposée.

D'autres méthodes connues visent à résoudre la problématique de détermination des propriétés radioélectriques d'un milieu à l'aide d'un système de détection radiofréquence. On peut citer notamment les demandes de brevets et brevets WO2020180191, EP3164672, WO2022091456, FR3041108 et FR3142553.

Toutes ces méthodes présentent des inconvénients, certaines méthodes sont invasives et destructives, d'autres nécessitent la connaissance préalable de la géométrie du milieu, d'autres ne sont pas applicables à un milieu inhomogène. De manière générale, ces méthodes proposent des solutions de traitement de données radar dans le domaine temporel.

Il existe un besoin pour une méthode permettant de caractériser précisément les propriétés radioélectriques d'un milieu au moyen d'un système de détection radiofréquences qui résout les limitations des méthodes de l'art antérieur. La méthode proposée doit notamment être faiblement complexe pour être compatible d'une mise en œuvre dans un système embarqué à ressources limitées.

Il est proposé une nouvelle méthode de caractérisation qui opère dans le domaine fréquentiel et qui présente une complexité diminuée par rapport aux méthodes de l'art antérieur.

A cet effet, l'invention a pour objet une méthode de détermination de propriétés radioélectriques d'un milieu comprenant les étapes de :

- Déterminer, au moyen d'un système de détection radiofréquence comprenant au moins un émetteur et un récepteur, pour chaque couple associant un récepteur et un émetteur, une mesure d'une fonction de transfert fréquentielle d'un canal de transmission caractérisant ledit milieu,

- Déterminer un modèle de ladite fonction de transfert fréquentielle fonction d'au moins une variable de position et d'au moins une variable caractérisant l'au moins une propriété radioélectrique du milieu,

- Déterminer une fonction d'estimation d'un coefficient de corrélation entre l'au moins une mesure d'une fonction de transfert fréquentielle et le modèle de ladite fonction,

- Fixer une valeur initiale de l'au moins une propriété radioélectrique,

- Rechercher un ensemble de premiers maxima locaux de la fonction d'estimation sur le domaine défini par l'au moins une variable de position pour la valeur initiale de l'au moins une propriété radioélectrique,

- Pour chaque premier maximum local, rechercher un second maximum local de la fonction d'estimation sur le domaine défini par l'au moins une variable caractérisant l'au moins une propriété radioélectrique du milieu et sur un domaine défini par l'au moins une variable de position contraint par le premier maximum local.

Selon un aspect particulier de l'invention, la recherche d'un second maximum local de la fonction d'estimation est réalisée sur un intervalle de l'au moins une variable de position au voisinage du premier maximum local.

Selon un aspect particulier de l'invention, la recherche d'un second maximum local de la fonction d'estimation est réalisée sur un domaine contraint de sorte que la longueur électrique, définie par le produit de la racine carrée de la partie réelle de la permittivité diélectrique par la distance totale parcourue par le signal entre un émetteur et un récepteur, soit sensiblement constante entre les premiers maxima locaux et le second maximum local.

Selon un aspect particulier de l'invention, la contrainte de longueur électrique sensiblement constante est imposée pour tous les couples associant un émetteur et un récepteur.

Selon un aspect particulier de l'invention, la contrainte de longueur électrique sensiblement constante est imposée seulement pour le couple associant un émetteur et un récepteur qui minimise la distance parcourue par un signal radiofréquence avec un diffuseur de coordonnées égales au premier maximum local.

Dans une variante de réalisation, la méthode selon l'invention comprend en outre une étape de déterminer une image du milieu en fonction de l'au moins une variable de position à partir des dites mesures et de l'au moins une valeur initiale, la recherche dudit ensemble de premiers maxima locaux étant réalisée sur l'image et non la fonction d'estimation.

Selon un aspect particulier de l'invention, les étapes de déterminer au moins une mesure de la fonction de transfert fréquentielle et de déterminer un modèle de ladite fonction de transfert sont réalisées pour chaque couple associant un émetteur et un récepteur du système de détection, la méthode comprenant en outre la détermination d'un coefficient de corrélation entre ladite mesure et ledit modèle pour chacun desdits couples, la fonction d'estimation étant calculée à partir de tous les coefficients de corrélation.

Selon un aspect particulier de l'invention, l'amplitude et la phase du modèle de la fonction de transfert fréquentielle dépendent de la distance entre une antenne d'émission et un point du plan et de la distance entre le point du plan et une antenne de réception.

Selon un aspect particulier de l'invention, l'au moins une variable caractérisant une propriété radioélectrique du milieu est prise parmi : la partie réelle ou la partie imaginaire de la permittivité diélectrique ou de la perméabilité.

Selon un aspect particulier de l'invention, le coefficient de corrélation est déterminé par une méthode d'égalisation de type ZF,MMSE ou MRC.

L'invention a aussi pour objet un dispositif de détermination de propriétés radioélectriques d'un milieu comprenant un système de détection radiofréquence comprenant au moins un émetteur et un récepteur et une unité de traitement, le système étant configuré pour mettre en œuvre la méthode selon l'invention.

Selon un aspect particulier de l'invention, le système de détection radiofréquence est un radar à pénétration de sol.

L'invention a aussi pour objet un programme d'ordinateur comprenant des instructions de code qui conduisent le dispositif selon l'invention à exécuter les étapes de la méthode selon l'invention ainsi qu'un support lisible par ordinateur, sur lequel est enregistré le programme d'ordinateur selon l'invention.

D'autres caractéristiques et avantages de la présente invention apparaîtront mieux à la lecture de la description qui suit en relation aux dessins annexés suivants.

[Fig. 1] représente un organigramme détaillant le principe général d'une méthode de détermination de propriétés radioélectriques d'un milieu selon l'invention,

[Fig. 2] schématise un exemple de système de détection radiofréquence, apte à mettre en œuvre l'invention,

[Fig. 3] illustre une représentation graphique d'un modèle de fonction de transfert,

[Fig. 4] représente un organigramme décrivant les étapes d'une méthode d'optimisation sous contraintes selon un premier mode de réalisation de l'invention,

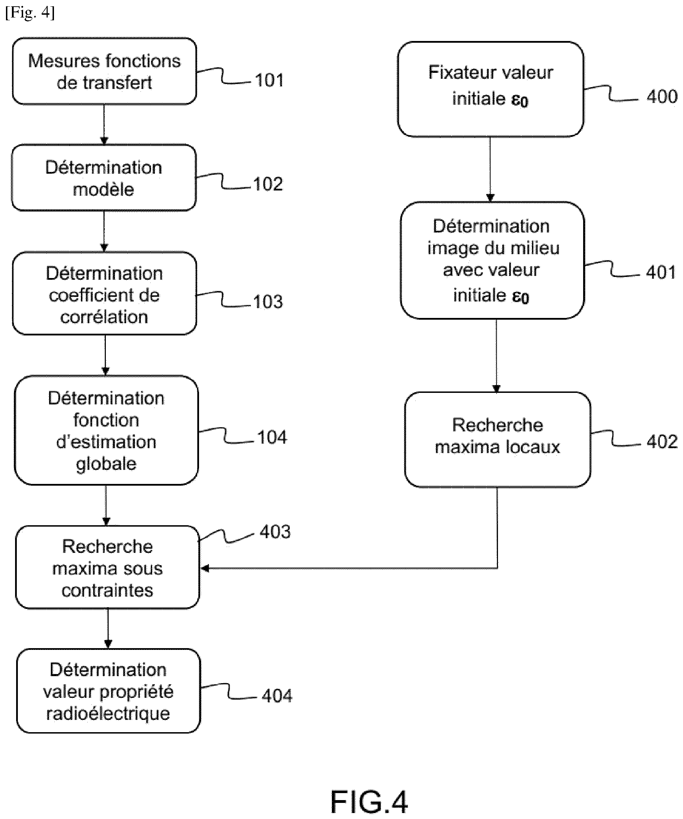

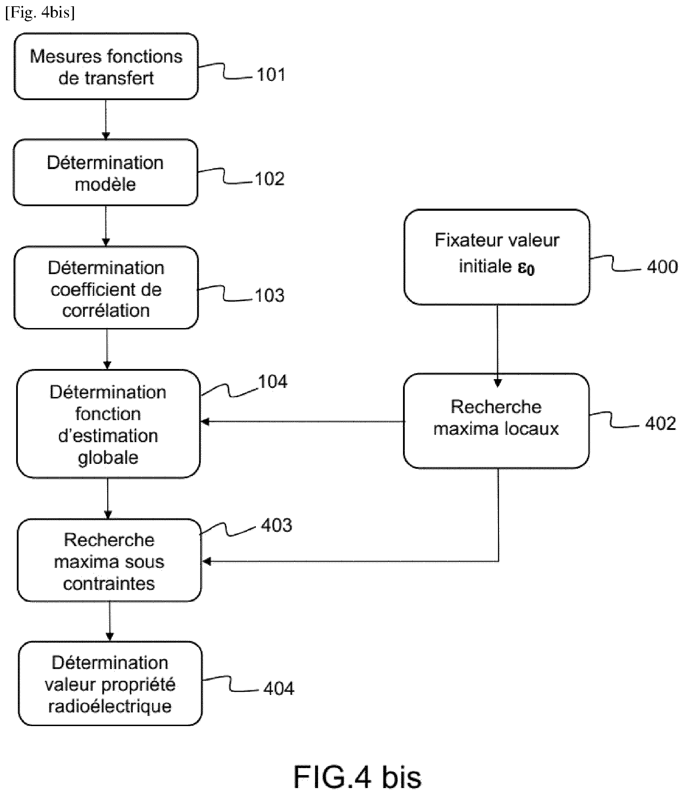

[Fig. 4bis] représente un organigramme décrivant les étapes d'une méthode d'optimisation sous contraintes selon un deuxième mode de réalisation de l'invention,

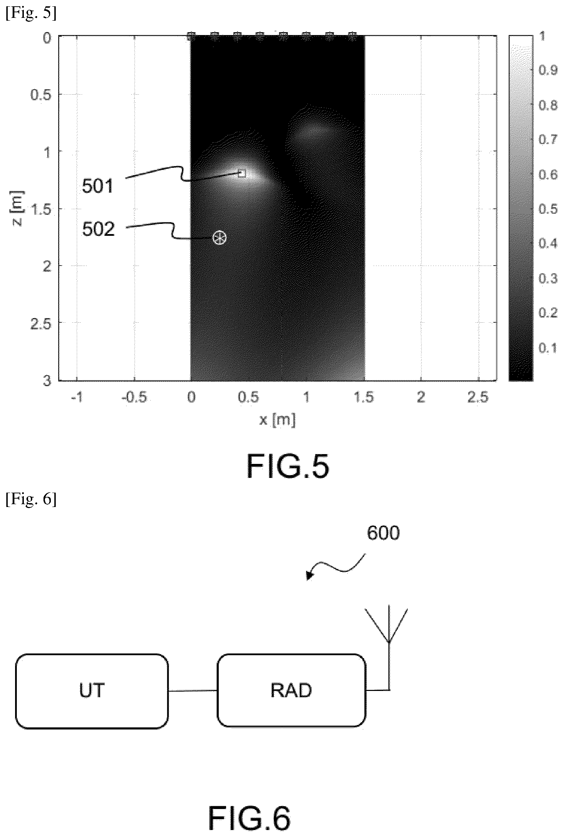

[Fig. 5] représente une image radar du sol dans le plan (x,z),

[Fig. 6] schématise un exemple de système apte à mettre en œuvre l'invention.

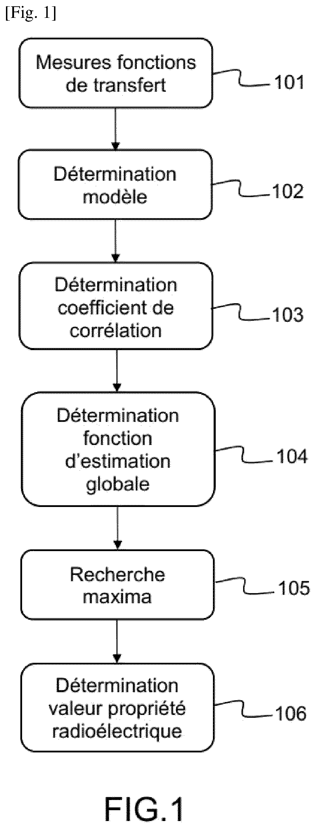

La figure 1 représente un organigramme décrivant le principe général d'une méthode de détermination de propriétés radioélectriques d'un milieu selon l'invention.

Dans la suite de la description, la méthode est décrite pour un exemple non limitatif destiné à la détermination de la permittivité d'un milieu tel que le sol au moyen d'un système de détection radiofréquences tel qu'un radar à pénétration de sol. Cependant l'invention n'est pas limitée à la détermination de la permittivité mais s'étend à la détermination de toute propriété radioélectrique caractérisant un milieu tel qu'également la perméabilité.

De même l'invention, n'est pas limitée à l'emploi d'un dispositif radar à pénétration de sol mais s'étend à tout dispositif de détection radiofréquence.

La méthode débute à l'étape 101 avec un ensemble de mesures de fonctions de transfert fréquentielles au moyen d'un dispositif de détection radiofréquence.

Le système de détection radiofréquence est composé d'un émetteur capable d'émettre un signal radiofréquence via au moins une antenne d'émission Tx et d'un récepteur capable de recevoir ce signal radiofréquence via au moins une antenne de réception Rx. Plusieurs versions du signal transmis sont observées et dépendent des positions de l'antenne Tx et de l'antenne Rx.

Selon une variante de réalisation, ces différentes versions du signal reçu sont obtenues à partir de plusieurs antennes Tx situées à des positions fixes et plusieurs antennes Rx situées à des positions fixes, selon une configuration du dispositif de détection radiofréquence appelée « Multiple Input Multiple Output (MIMO) ».

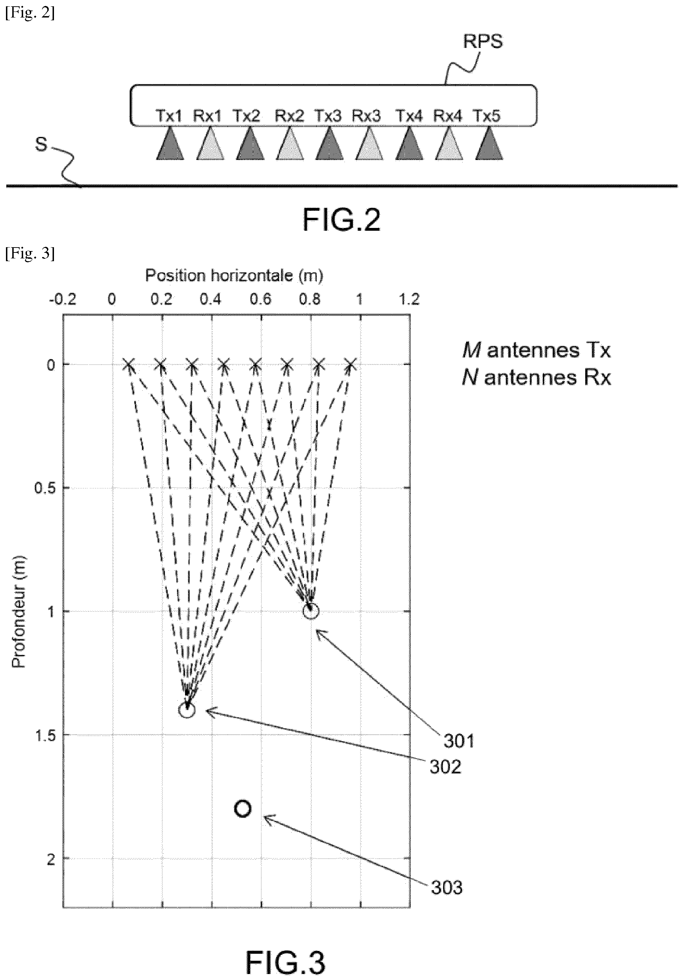

La figure 2 schématise un exemple d'un tel système de détection radiofréquence sous la forme d'un radar à pénétration de sol RPS ayant cinq antennes en émission et quatre antennes en réception.

Alternativement les différentes mesures peuvent également être obtenues en réalisant des transmissions successives, et en déplaçant les antennes Tx ou Rx entre chaque transmission.

Ainsi, on obtient une mesure pour chaque couple associant une antenne d'émission Tx à une antenne de réception Rx pour une position donnée de ces deux antennes.

L'invention s'applique également au cas où une seule antenne sert à la fois en émission et en réception et est connectée à l'émetteur et au récepteur par l'intermédiaire d'un dispositif de séparation tel qu'un coupleur.

La séparation entre les signaux transmis via chaque paire d'antennes Tx et Rx est réalisée dans le domaine temporel à travers des transmissions successives, mais elle peut également être réalisée par toute méthode d'accès multiple classique, comme la séparation en fréquence ou par codes.

On se place à présent dans le cas général d'un système composé de M antennes en émission et N antennes en réception ou plus généralement de M positions d'antennes différentes en émission et N positions d'antennes différentes en réception. M et N sont deux entiers strictement positifs.

Le système de détection radiofréquences comporte en outre un module d'estimation configuré pour déterminer, à partir du signal transmis via l'antenne Tx en position m et reçu via l'antenne Rx en position n, une fonction de transfert complexe $H_{mn}(f)$ pour différentes valeurs de fréquences f.

Plusieurs méthodes d'obtention de la fonction de transfert $H_{mn}(f)$ et de choix d'un signal à transmettre existent dans l'état de l'art et sont des techniques connues du domaine de l'estimation du canal ou du sondage radioélectrique. Les références [8], [9], [10] donnent des exemples de telles méthodes.

La fonction de transfert $H_{mn}(f)$ dépend de la composition du milieu traversé par le signal, et en particulier de la répartition spatiale des propriétés radioélectriques du milieu, et dans le cas de la présence de cibles, de la position et de la Surface Equivalente Radar (SER) des cibles.

A l'issue de l'étape 101, on a donc une mesure de fonction de transfert $H_{mn}(f)$ pour plusieurs valeurs de fréquences et pour chaque couple (m,n) associant une antenne en émission et une antenne en réception pour lesquelles une mesure a été réalisée.

A l'étape 102, on détermine ensuite un modèle théorique de la fonction de transfert fréquentielle qui prend en compte les propriétés radioélectriques du milieu traversé par le signal.

Par exemple, le modèle choisi est adapté à un diffuseur ponctuel situé à une position $r_p$ dans un milieu homogène de permittivité de partie réelle $\varepsilon'_r$ et de partie imaginaire négligeable. La fonction de transfert entre l'émetteur m et le récepteur n est modélisée par l'équation (1) suivante :

$$W_{mn}(f) = \frac{\sqrt{G_m G_n}.c}{f.\|r_p - r_m\|.\|r_n - r_p\|.(4\pi)^{3/2}.\sqrt{\varepsilon'_r}} . e^{-i2\pi f \frac{\sqrt{\varepsilon'_r}}{c}(\|r_p - r_m\| + \|r_n - r_p\|)} \quad (1)$$

$r_m$ et $r_n$ sont respectivement les positions de l'antenne d'émission et de l'antenne de réception,

$G_m$ et $G_n$ sont les gains respectifs des antennes d'émission et de réception,

C'est la vitesse de la lumière dans le vide.

Le modèle donné par l'équation (1) considère d'une part le déphasage lié à la géométrie de la scène pour une propagation rectiligne et d'autre part une amplitude donnée par l'équation radar issue de la littérature (voir référence [7]).

La figure 3 schématise une représentation graphique du modèle de l'équation (1) pour M=N=8 et pour deux diffuseurs 301,302. La référence 303 représente un point test situé aux coordonnées (x,z).

Sans sortir du cadre de l'invention, d'autres modèles peuvent être développés en remplacement de celui de l'équation (1) dans la mesure où ils prennent en compte au moins une propriété radioélectrique du milieu, par exemple la

permittivité $\varepsilon$'r.

Par exemple, l'équation (1) peut être remplacée par l'équation (1bis) suivante dans laquelle le coefficient $\sqrt{\varepsilon'_r}$ est remplacé par $\varepsilon'_r$.

$$W_{mn}(f) = \frac{\sqrt{G_m G_n}.c}{f.\|r_p - r_m\|.\|r_n - r_p\|.(4\pi)^{3/2}.\varepsilon'_r} . e^{-i2\pi f \frac{\sqrt{\varepsilon'_r}}{c}(\|r_p - r_m\| + \|r_n - r_p\|)} \quad (1bis)$$

En effet, l'équation (1) fournit un modèle de fonction de transfert adapté à un diffuseur ponctuel situé à une position $r_p$ dans un milieu homogène de permittivité de partie réelle $\varepsilon'_r$ et de partie imaginaire négligeable. Sa phase est calculée en considérant le retard accumulé par l'onde pour une propagation rectiligne entre l'antenne Tx et le diffuseur, puis entre le diffuseur et l'antenne Rx. Son amplitude est fournie par l'équation radar.

L'observation de résultats expérimentaux tend à montrer que le modèle donné par l'équation (1) n'est pas toujours optimal concernant la modélisation de l'amplitude.

Une explication physique à l'adaptation proposée par l'équation (1bis) est que la surface équivalente radar (SER) est supposée indépendante des propriétés radioélectriques du milieu. Or on estime que la SER d'un objet donné évolue de manière inversement proportionnelle à la permittivité $\varepsilon'_r$ du milieu.

Le module de l'équation (1bis) permet d'améliorer la focalisation, tel que cela est explicité par la suite.

A l'étape 103, on détermine un coefficient de corrélation entre la mesure réalisée à l'étape 101 et le modèle déterminé à l'étape 102, pour chaque couple d'antennes.

Par exemple, le coefficient de corrélation est déterminé au moyen de la relation (2) qui représente une égalisation de type Zero Forcing (ZF).

$$\mathcal{L}_{mn}(f, r_p, \varepsilon'_r) = \frac{H_{mn}(f).W_{mn}{}^*(f, r_p, \varepsilon'_r)}{|W_{mn}(f, r_p, \varepsilon'_r)|^2} \quad (2)$$

\* représente l'opérateur de conjugaison complexe

Alternativement, le coefficient de corrélation peut être obtenu via une autre formule d'égalisation, comme par exemple celles de type « Minimum Mean Square Error » (MMSE) pour « Erreur quadratique moyenne minimale » en français ou « Maximum Ratio Combining » (MRC) pour « Combinaison à rapport maximal » en français

Le coefficient de corrélation dépend au moins de la fréquence, d'au moins une coordonnée d'espace et d'au moins une propriété radioélectrique et est représentatif d'une corrélation entre la fonction de transfert mesurée $H_{mn}(f)$ et le modèle $W_{mn}(f, r_p, \varepsilon'_r)$.

Les étapes 101,102,103 sont itérées pour chaque couple associant une antenne en émission d'indice m et une antenne en réception d'indice n.

A l'étape 104, on détermine ensuite une fonction d'estimation globale du coefficient de corrélation pour l'ensemble des observations réalisées par le système de détection radiofréquence.

En considérant que les fonctions de transfert sont observées sur un ensemble de L fréquences discrètes $f_l$, ainsi que pour les différents angles bi-statiques provenant de M positions $r_m$ de l'émetteur et N positions $r_n$ du récepteur, la fonction d'estimation globale est obtenue en effectuant une somme cohérente des différentes observations tel que représenté par l'équation (3) :

$$\mathcal{L}(r_p, \varepsilon'_r) = \frac{1}{MNL} \sum_{m=1}^{M} \sum_{n=1}^{N} \sum_{l=1}^{L} \mathcal{L}_{mn}(f_l, r_p, \varepsilon'_r) \quad (3)$$

A l'étape 105, on recherche ensuite au moins un maximum de la fonction d'estimation globale ou plus précisément de sa valeur absolue ou de sa norme au carré lorsque cette fonction est complexe. La recherche est effectuée dans l'espace multidimensionnel constitué des variables d'espace et des variables caractérisant les propriétés radioélectriques formant le domaine de définition de cette fonction.

Cette approche repose sur le principe que les valeurs de propriétés radioélectriques qui correspondent le mieux à la réalité physique du terrain fournissent des valeurs de coefficient de corrélation qui sont cohérentes entre différentes fréquences et différentes configurations d'observations, maximisant le module de la somme cohérente de l'équation (3).

Cette maximisation du module de la somme cohérente des coefficients de corrélation pour des valeurs de variables qui correspondent à une réalité physique observée est encore appelé « principe de focalisation ».

A l'étape 106 de la méthode, on déduit au moins une valeur de propriété radioélectrique associée à une position spatiale.

Par exemple, pour l'exemple des équations (2) et (3), on obtient une valeur de position $r_p^{max}$ et une valeur de permittivité diélectrique $\varepsilon'^{max}_r$ associée à cette position qui sont définis par l'équation (4) :

$$\left(r_p^{max}, \varepsilon'^{max}_r\right) = \underset{r_p, \varepsilon'_r}{\operatorname{argmax}}\left|\mathcal{L}\left(r_p, \varepsilon'_r\right)\right| \quad (4)$$

Argmax représente la fonction mathématique argument du maximum, qui fournit les valeurs des variables pour lesquelles une fonction atteint son maximum.

Etant donné le modèle a priori donné par l'équation (1), le vecteur $r_p^{max}$ renseigne sur la position du diffuseur ponctuel fournissant la Surface Equivalente Radar la plus importante dans l'espace observé et $\varepsilon'^{max}_r$ renseigne sur la valeur la plus représentative de la partie réelle de la permittivité diélectrique moyenne dans la partie du milieu parcourue par l'onde électromagnétique entre les antennes d'émission et les antennes de réception via la position $r_p^{max}$.

Un inconvénient de la méthode décrite à la figure 1 réside dans la complexité de résolution lié à l'espace de recherche multidimensionnel. En effet, lorsque l'espace des dimensions est en 3D, l'espace de recherche total est à 4 dimensions en comptant la recherche de la partie réelle de la permittivité diélectrique. Sans contraintes a priori sur les intervalles de recherche de ces différentes variables, l'algorithme décrit à la figure 1 est complexe à mettre en œuvre.

Pour pallier cet inconvénient, il est proposé une méthode d'optimisation sous contraintes pour limiter les espaces de recherche.

Cette méthode est présentée à la figure 4 pour le cas de la détermination de la partie réelle de la permittivité d'un milieu, par exemple le sol. L'invention s'applique à l'identique à d'autres variables de caractérisation du milieu telles que la perméabilité.

Les étapes 101 à 104 sont identiques à celles déjà décrites à la figure 1.

En parallèle ou successivement, à l'étape 400, on fixe une valeur initiale a priori de la partie réelle de la permittivité, notée $\varepsilon_0$. Pour cette valeur, on détermine (étape 401) une image en 2D ou 3D du milieu au moyen d'une méthode d'imagerie radar de l'art antérieur telle que la méthode du diffuseur ponctuel. Un exemple d'une telle méthode est décrit dans la demande de brevet du Demandeur FR3124607.

Par exemple, si le milieu visé est le sol, l'image est réalisée en 2D dans le plan (x,z). Un exemple d'une telle image est donné à la figure 5.

A l'étape 402, on recherche dans l'image l'ensemble des maxima locaux qui sont regroupés dans un ensemble $\Omega$ = $\{x_i, z_i\}_{i=1\ldots P}$, P étant le nombre des maxima locaux.

Par exemple, sur la figure 5, on a représenté un exemple de maximum local 501 détecté à l'étape 402 pour une valeur initiale de permittivité $\varepsilon_0$=0.

Dans une variante de réalisation de l'invention décrite à la figure 4bis, l'étape 402 est effectuée non pas en recherchant les maxima locaux sur une image radar mais en recherchant directement les maxima locaux de la fonction d'estimation globale déterminée à l'étape 104, avec la valeur de permittivité fixée à sa valeur initiale à l'étape 400. Dans cette variante, l'étape 401 de détermination d'une image du milieu est supprimée.

A l'étape 403, on recherche ensuite les valeurs de x,z et $\varepsilon$ qui maximisent la fonction d'estimation globale déterminée à l'étape 104 en fixant les intervalles de recherche suivants :

$$(x, y, z) = max\|\mathcal{L}(x, z, \varepsilon)\|^2$$

$$x \in [x_i - \delta_x; x_i + \delta_x]$$

$$z \in [z_i - \delta_z; z_i + \delta_z]$$

$\varepsilon \in [1; \varepsilon_{max}]$
$\delta_x$, $\delta_z$ et $\varepsilon_{max}$ délimitent les intervalles de recherche.

L'étape 403 est exécutée pour chaque maxima local de l'ensemble $\Omega$

Dans un mode de réalisation particulier, la fonction d'estimation globale est donnée par la relation :

$$\mathcal{L}(x, z, \varepsilon) = \frac{1}{MNL} \sum_{m=1}^{M} \sum_{n=1}^{N} \sum_{l=1}^{L} \mathcal{L}_{mn}(f_l, x, z, \varepsilon)$$

$$\mathcal{L}_{mn}(f, x, z, \varepsilon) = \frac{H_{mn}(f)}{\varepsilon f d_{mn}^*(x,z)} e^{-i2\pi f \frac{\sqrt{\varepsilon}}{c} d_{mn}(x,z)} \quad (5)$$

$$d_{mn}(x, z) = \sqrt{(x - x_m)^2 + (z - z_m)^2} + \sqrt{(x - x_n)^2 + (z - z_n)^2}$$

$$d_{mn}^*(x, z) = \sqrt{(x - x_m)^2 + (z - z_m)^2} \times \sqrt{(x - x_n)^2 + (z - z_n)^2}$$

$d_{mn}(x, z)$ est la somme des distances respectives entre le point de coordonnées (x,z) et respectivement l'antenne d'émission m et l'antenne de réception n du radar. $d_{mn}^*(x, z)$ est le produit des distances respectives entre le point de coordonnées (x,z) et respectivement l'antenne d'émission m et l'antenne de réception n du radar.

La méthode décrite à la figure 4 présente l'avantage de limiter l'espace de recherche puisque la recherche de maxima dans l'espace de localisation spatiale est limitée à des intervalles de voisinage autour des positions détectées à l'étape 402 sur l'image du milieu.

Cependant, il est encore nécessaire de fixer les bornes des intervalles de recherche autour de ces positions.

Une variante de réalisation de la méthode consiste à exploiter le concept de longueur électrique définie par la relation : $c(m, n) = \sqrt{\varepsilon} d_{mn}(x, z)$.

En théorie, la longueur électrique associée à un maximum local de l'image du milieu doit être constante quelle que soit la position x, z du maximum local obtenu avec différentes valeurs a priori de permittivité diélectrique.

On peut ainsi exploiter cette propriété pour limiter l'espace de recherche des valeurs de x et z tels qu'elles respectent cette propriété :

$$c_i(m, n) = \sqrt{\varepsilon_0} d_{mn}(x_i, z_i) = \sqrt{\varepsilon} d_{mn}(x, z) \quad (6)$$

L'équation (6) doit être respectée pour tous les maxima locaux $(x_i, z_i)$ détectés à l'étape 402 et pour tous les indices m,n parcourant les positions respectives des antennes d'émission et de réception.

Ainsi, l'équation (6) fixe M x N contraintes pour chaque maxima local détecté à l'étape 402, M étant le nombre d'émetteurs et N le nombre de récepteurs. Ces contraintes permettent de réduire l'espace de recherche des trois variables (x,z,$\varepsilon$). L'optimisation peut également se réduire à la recherche de deux variables puisque l'une des trois variables est liée aux deux autres par l'équation (6).

Dans une autre variante de réalisation de l'invention, on prend en compte l'impact du bruit de mesure sur la valeur de la longueur électrique. En effet, l'estimation de la longueur électrique $c_i(m, n)$ peut-être bruitée. Pour prendre en compte cette incertitude, on ajoute une contrainte suivante sous la forme des équations :

$$\alpha c_i(m, n) - \sqrt{\varepsilon} d_{mn}(x, z) = 0, \forall n, \forall m \quad (7)$$

$$\alpha \in [1 - \delta_\alpha; 1 + \delta_\alpha]$$

$\delta_\alpha$ définit l'intervalle de variation du paramètre $\alpha$.

Dans encore une autre variante de réalisation, afin de limiter encore le domaine de recherche des variables, l'équation (6) est appliquée uniquement pour la paire d'antennes d'émission et de réception qui minimise la distance $d_{mn}(x_i, z_i)$. Cela permet encore de diminuer l'espace de recherche des variables.

Bien que l'exemple ci-dessus ait été décrit pour une localisation dans l'espace 2D (x,z), la méthode s'applique à l'identique dans l'espace 3D (x,y,z). On ajoute alors des contraintes sur la variable y.

La recherche de maxima de la fonction d'estimation globale est réalisée au moyen d'algorithmes d'optimisation numérique tels que décrits par exemple dans la référence [11].

La figure 5 montre un exemple de résultat obtenu par application de la méthode selon l'invention.

Une cible est placée en (x = 0,254; z = 1,758) dans un milieu homogène de permittivité $\varepsilon$ = 4,255. Une autre cible est placée dans le milieu pour apporter une perturbation. La seconde cible est en position (1,207; 1,080). Sa puissance rayonnée est quatre fois plus faible que la première cible.

La première détection (étape 402) est réalisée avec $\varepsilon_0$ = 9. Un maximum local en position ($x_{i=1}$ = 0,440; $z_{i=1}$ = 1,200) est identifié (référence 501). On applique alors l'étape 403 de la méthode et on obtient un maxima 502 en position (x = 0,252; z = 1,761; $\varepsilon$ = 4,242) soit une erreur inférieure au millimètre pour la position et de l'ordre de 0,01 pour la permittivité.

La figure 6 représente schématiquement un système 600 de détermination de propriétés radioélectriques d'un milieu selon un mode de réalisation de l'invention.

Le système 600 comporte principalement un dispositif de détection radiofréquences RAD du type de celui décrit en figure 2 et une unité de traitement UT configurée pour mettre en œuvre la méthode selon l'invention à partir de mesures réalisées par le dispositif RAD.

L'unité de traitement UT peut être réalisée sous forme logicielle et/ou matérielle notamment en utilisant un ou plusieurs processeur(s) et une ou plusieurs mémoire(s). Le processeur peut être un processeur générique, un processeur spécifique, un circuit intégré propre à une application (connu aussi sous le nom anglais d'ASIC pour « Application-Specific Integrated Circuit ») ou un réseau de portes programmables in situ (connu aussi sous le nom anglais de FPGA pour « Field-Programmable Gate Array »).

Le système 600 peut comporter une interface utilisateur pour afficher des résultats produits par la méthode.

Références

[1] S. Serkan and V. Borecky, "Estimation methods for obtaining GPR signal velocity," in Proceedings of the Third International Conference on ACSEE, Zurich, Switzerland, 2015, pp. 10-11.

[2] E. Forte and M. Pipan, "Review of multi-offset GPR applications : Data acquisition, processing and analysis," Signal processing, vol. 132, pp. 210-220, 2017.

[3] I. Dal Bo, A. Klotzsche et al., "Geophysical imaging of regolith in landscapes along a climate and vegetation gradient in the Chilean coastal cordillera," Catena, vol. 180, pp. 146-159, 2019.

[4] Q. Dou, L. Wei, D. R. Magee, and A. G. Cohn, "Real-time hyperbola recognition and fitting in GPR data," IEEE Transactions on Geoscience and Remote Sensing, vol. 55, no. 1, pp. 51-62, 2017.

[5] C. Ozdemir, S. Demirci et al., "A review on migration methods in B-scan ground penetrating radar imaging," Mathematical Problems in Engineering, vol. 2014, 2014.

[6] Z. Dong, X. Feng et al., "3D migration depth focus velocity analysis of hand-held ground penetrating radar," Geosciences, vol. 12, no. 4, p. 178, 2022.

[7] E. F. Knott, J. F. Schaeffer, and M. T. Tulley, Radar Cross Section. SciTech Publishing, 2004.

[8] Demery, D. A., J. D. Parsons, and A. M. D. Turkmani. "Sounding techniques for wideband mobile radio channels: a review." IEEE Proceedings (Communications, Speech and Vision) 138.5 (1991): 437-446.

[9] Laurenson, Dave, and Peter Grant. "A review of radio channel sounding techniques." 2006 14th European Signal Processing Conference. IEEE, 2006.

[10] Ullah, M. Habib, and A. Unggul Priantoro. "A review on multiplexing schemes for MIMO channel sounding." International Journal of Computer Science and Network Security 9.6 (2009): 294-300.

[11] Michael Grant and Stephen Boyd. CVX: Matlab software for disciplined convex programming, version 2.0 beta. https://cvxr.com/cvx, September 2013

**Revendications**

1. Méthode, mise en œuvre par ordinateur, de détermination de propriétés radioélectriques d'un milieu comprenant les étapes de :

   - Déterminer (101), au moyen d'un système de détection radiofréquence comprenant au moins un émetteur et un récepteur, pour chaque couple associant un récepteur et un émetteur, une mesure d'une fonction de transfert fréquentielle d'un canal de transmission caractérisant ledit milieu,
   - Déterminer (102) un modèle de ladite fonction de transfert fréquentielle fonction d'au moins une variable de position et d'au moins une variable caractérisant l'au moins une propriété radioélectrique du milieu,
   - Déterminer (103,104) une fonction d'estimation d'un coefficient de corrélation entre l'au moins une mesure d'une fonction de transfert fréquentielle et le modèle de ladite fonction,
   - Fixer (400) une valeur initiale de l'au moins une propriété radioélectrique,
   - Rechercher (402) un ensemble de premiers maxima locaux de la fonction d'estimation sur le domaine défini par l'au moins une variable de position pour la valeur initiale de l'au moins une propriété radioélectrique,
   - Pour chaque premier maximum local, rechercher (403) un second maximum local de la fonction d'estimation sur le domaine défini par l'au moins une variable caractérisant l'au moins une propriété radioélectrique du milieu et sur un domaine défini par l'au moins une variable de position contraint par le premier maximum local.

2. Méthode de détermination de propriétés radioélectriques d'un milieu selon la revendication 1 dans laquelle la recherche (403) d'un second maximum local de la fonction d'estimation est réalisée sur un intervalle de l'au moins une variable de position au voisinage du premier maximum local.

3. Méthode de détermination de propriétés radioélectriques d'un milieu selon la revendication 1 dans laquelle la recherche (403) d'un second maximum local de la fonction d'estimation est réalisée sur un domaine contraint de sorte que la longueur électrique, définie par le produit de la racine carrée de la partie réelle de la permittivité diélectrique par la distance totale parcourue par le signal entre un émetteur et un récepteur, soit sensiblement constante entre les premiers maxima locaux et le second maximum local.

4. Méthode de détermination de propriétés radioélectriques d'un milieu selon la revendication 3 dans laquelle la contrainte de longueur électrique sensiblement constante est imposée pour tous les couples associant un émetteur et un récepteur.

5. Méthode de détermination de propriétés radioélectriques d'un milieu selon la revendication 3 dans laquelle la contrainte de longueur électrique sensiblement constante est imposée seulement pour le couple associant un émetteur et un récepteur qui minimise la distance parcourue par un signal radiofréquence avec un diffuseur de coordonnées égales au premier maximum local.

6. Méthode de détermination de propriétés radioélectriques d'un milieu selon l'une quelconque des revendications précédentes comprenant en outre une étape de déterminer (401) une image du milieu en fonction de l'au moins une variable de position à partir des dites mesures et de l'au moins une valeur initiale, la recherche (402) dudit ensemble de premiers maxima locaux étant réalisée sur l'image et non la fonction d'estimation.

7. Méthode de détermination de propriétés radioélectriques d'un milieu selon l'une quelconque des revendications précédentes dans laquelle les étapes de déterminer au moins une mesure de la fonction de transfert fréquentielle et de déterminer un modèle de ladite fonction de transfert sont réalisées pour chaque couple associant un émetteur et un récepteur du système de détection, la méthode comprenant en outre la détermination (103) d'un coefficient de corrélation entre ladite mesure et ledit modèle pour chacun desdits couples, la fonction d'estimation étant calculée à partir de tous les coefficients de corrélation.

8. Méthode de détermination de propriétés radioélectriques d'un milieu selon la revendication 7 dans laquelle l'amplitude et la phase du modèle de la fonction de transfert fréquentielle dépendent de la distance entre une antenne d'émission et un point du plan et de la distance entre le point du plan et une antenne de réception.

9. Méthode de détermination de propriétés radioélectriques d'un milieu selon l'une quelconque des revendications précédentes dans laquelle l'au moins une variable caractérisant une propriété radioélectrique du milieu est prise parmi : la partie réelle ou la partie imaginaire de la permittivité diélectrique ou de la perméabilité.

**10.** Méthode de détermination de propriétés radioélectriques d'un milieu selon l'une quelconque des revendications précédentes dans laquelle le coefficient de corrélation est déterminé par une méthode d'égalisation de type ZF,MMSE ou MRC.

**11.** Dispositif (600) de détermination de propriétés radioélectriques d'un milieu comprenant un système de détection radiofréquence (RAD) comprenant au moins un émetteur et un récepteur et une unité de traitement (UT), le système étant configuré pour mettre en œuvre la méthode selon l'une quelconque des revendications précédentes.

**12.** Dispositif selon la revendication 11 dans lequel le système de détection radiofréquence est un radar à pénétration de sol.

**13.** Programme d'ordinateur comprenant des instructions de code qui conduisent le dispositif selon l'une quelconque des revendications 11 ou 12 à exécuter les étapes de la méthode selon l'une quelconque des revendications 1 à 10.

**14.** Support lisible par ordinateur, sur lequel est enregistré le programme d'ordinateur selon la revendication 13.

[Fig. 1]

```
┌─────────────────────┐
│   Mesures fonctions  │──── 101
│    de transfert      │
└─────────────────────┘
           │
           ▼
┌─────────────────────┐
│    Détermination     │──── 102
│      modèle          │
└─────────────────────┘
           │
           ▼
┌─────────────────────┐
│    Détermination     │
│   coefficient de     │──── 103
│     corrélation      │
└─────────────────────┘
           │
           ▼
┌─────────────────────┐
│    Détermination     │
│      fonction        │
│    d'estimation      │──── 104
│      globale         │
└─────────────────────┘
           │
           ▼
┌─────────────────────┐
│     Recherche        │──── 105
│      maxima          │
└─────────────────────┘
           │
           ▼
┌─────────────────────┐
│    Détermination     │
│  valeur propriété    │──── 106
│   radioélectrique    │
└─────────────────────┘
```

# FIG.1

[Fig. 2]

FIG.2

[Fig. 3]

FIG.3

[Fig. 4]

FIG.4

[Fig. 4bis]

```
┌─────────────────────┐
│  Mesures fonctions  │╮___ 101
│    de transfert     │
└─────────────────────┘
          ↓
┌─────────────────────┐
│    Détermination    │╮___ 102
│       modèle        │
└─────────────────────┘
          ↓
┌─────────────────────┐        ┌─────────────────────┐
│    Détermination    │        │   Fixateur valeur   │
│   coefficient de    │╮___ 103│   initiale ε₀       │╮___ 400
│     corrélation     │        └─────────────────────┘
└─────────────────────┘                  ↓
          ↓           ___ 104  ┌─────────────────────┐
┌─────────────────────┐        │     Recherche       │
│    Détermination    │        │   maxima locaux     │╮___ 402
│      fonction       │←───────│                     │
│    d'estimation     │        └─────────────────────┘
│      globale        │
└─────────────────────┘
          ↓           ___ 403
┌─────────────────────┐
│     Recherche       │
│   maxima sous       │←───────────────────
│   contraintes       │
└─────────────────────┘
          ↓
┌─────────────────────┐
│    Détermination    │
│  valeur propriété   │╮___ 404
│   radioélectrique   │
└─────────────────────┘
```

FIG.4 bis

[Fig. 5]

FIG.5

[Fig. 6]

FIG.6

Europäisches
Patentamt

European
Patent Office

Office européen
des brevets

**RAPPORT DE RECHERCHE EUROPEENNE**

Numéro de la demande

EP 25 20 3787

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| X,D | US 2024/176012 A1 (MAKHOUL GLORIA [FR] ET AL) 30 mai 2024 (2024-05-30)<br>* figures 1,2 *<br>* alinéas [0002] - [0046] *<br>* alinéas [0065], [0073] *<br>* alinéas [0107] - [0129] * | 1-14 | INV.<br>G01V3/12<br>G01S13/88<br>G01V3/15<br>G01V3/38 |
| A | US 2023/266461 A1 (D'ERRICO RAFFAELE [FR] ET AL) 24 août 2023 (2023-08-24)<br>* le document en entier * | 1-14 | |
| A,D | WO 2022/091456 A1 (MITSUBISHI ELECTRIC CORP [JP] ET AL.) 5 mai 2022 (2022-05-05)<br>* le document en entier * | 1-14 | |
| A | LAMBOT SÉBASTIEN ET AL: "Analysis of air-launched ground-penetrating radar techniques to measure the soil surface water content",<br>WATER RESOURCES RESEARCH.<br>,<br>vol. 42, no. 11<br>1 novembre 2006 (2006-11-01), XP093261856, US<br>ISSN: 0043-1397, DOI: 10.1029/2006WR005097<br>Extrait de l'Internet:<br>URL:https://api.wiley.com/onlinelibrary/tdm/v1/articles/10.1029%2F2006WR005097<br>* le document en entier * | 1-14 | |

| | DOMAINES TECHNIQUES RECHERCHES (IPC) |
|---|---|
| | G01V<br>G01S |

Le présent rapport a été établi pour toutes les revendications

| Lieu de la recherche | Date d'achèvement de la recherche | Examinateur |
|---|---|---|
| La Haye | 20 janvier 2026 | Parmentier, Hélène |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

........................................................................
& : membre de la même famille, document correspondant

EPO FORM 1503 03.82 (P04C02)

# EP 4 722 765 A1

## ANNEXE AU RAPPORT DE RECHERCHE EUROPEENNE
## RELATIF A LA DEMANDE DE BREVET EUROPEEN NO.

EP 25 20 3787

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

20-01-2026

| Document brevet cité au rapport de recherche | Date de publication | Membre(s) de la famille de brevet(s) | Date de publication |
|---|---|---|---|
| US 2024176012 A1 | 30-05-2024 | EP 4375663 A1 | 29-05-2024 |
| | | FR 3142553 A1 | 31-05-2024 |
| | | US 2024176012 A1 | 30-05-2024 |
| US 2023266461 A1 | 24-08-2023 | EP 4172655 A1 | 03-05-2023 |
| | | FR 3111994 A1 | 31-12-2021 |
| | | US 2023266461 A1 | 24-08-2023 |
| | | WO 2022002700 A1 | 06-01-2022 |
| WO 2022091456 A1 | 05-05-2022 | AUCUN | |

EPO FORM P0460

Pour tout renseignement concernant cette annexe : voir Journal Officiel de l'Office européen des brevets, No.12/82

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2020180191 A
- EP 3164672 A
- WO 2022091456 A
- FR 3041108
- FR 3142553
- FR 3124607

**Littérature non-brevet citée dans la description**

- **S. SERKAN** ; **V. BORECKY**. Estimation methods for obtaining GPR signal velocity. *Proceedings of the Third International Conference on ACSEE, Zurich, Switzerland*, 2015, 10-11
- **E. FORTE** ; **M. PIPAN**. Review of multi-offset GPR applications : Data acquisition, processing and analysis. *Signal processing*, 2017, vol. 132, 210-220
- **I. DAL BO** ; **A. KLOTZSCHE et al.** Geophysical imaging of regolith in landscapes along a climate and vegetation gradient in the Chilean coastal cordillera. *Catena*, 2019, vol. 180, 146-159
- **Q. DOU** ; **L. WEI** ; **D. R. MAGEE** ; **A. G. COHN**. Real-time hyperbola recognition and fitting in GPR data. *IEEE Transactions on Geoscience and Remote Sensing*, 2017, vol. 55 (1), 51-62
- **C. OZDEMIR** ; **S₂. DEMIRCI et al.** A review on migration methods in B-scan ground penetrating radar imaging. *Mathematical Problems in Engineering*, 2014, vol. 2014
- **Z. DONG** ; **X. FENG et al.** 3D migration depth focus velocity analysis of hand-held ground penetrating radar. *Geosciences*, 2022, vol. 12 (4), 178
- **E. F. KNOTT** ; **J. F. SCHAEFFER** ; **M. T. TULLEY**. Radar Cross Section. SciTech Publishing, 2004
- **DEMERY, D. A.** ; **J. D. PARSONS** ; **A. M. D. TURKMANI**. Sounding techniques for wideband mobile radio channels: a review. *IEEE Proceedings (Communications, Speech and Vision)*, 1991, vol. 138.5, 437-446
- A review of radio channel sounding techniques.. **LAURENSON, DAVE** ; **PETER GRANT**. 2006 14th European Signal Processing Conference.. IEEE, 2006
- **ULLAH, M. HABIB** ; **A. UNGGUL PRIANTORO**. A review on multiplexing schemes for MIMO channel sounding. *International Journal of Computer Science and Network Security*, 2009, vol. 9.6, 294-300
- **MICHAEL GRANT** ; **STEPHEN BOYD**. *CVX: Matlab software for disciplined convex programming, version 2.0 beta*, September 2013, https://cvxr.com/cvx